# FASCICULE DE BREVET EUROPEEN

(11) **EP 0 752 893 B1**
(45) Date de publication et mention de la délivrance du brevet: **07.07.1999**
(21) Numéro de dépôt: 94915190.6
(22) Date de dépôt: 29.04.1994
(51) Int. Cl.: A61M 1/00

(54) **APPAREIL A MOUCHER LES PERSONNES**
NASENPUTZVORRICHTUNG
DEVICE FOR REMOVING NASAL SECRETIONS

(30) Priorité: 04.05.1993 FR 9305668
(43) Date de publication de la demande: 15.01.1997
(73) Titulaire: INCOMEX, Monaco (MC)
(72) Inventeur: Valette, Jackie, 42450 Sury-le-Comtal (FR)
(74) Mandataire: Thivillier, Patrick
(86) Numéro de dépôt international: FR9400492
(87) Numéro de publication internationale: WO9425083

(56) Documents cités:
- EP-A- 0 451 062
- FR-A- 2 572 938
- GB-A- 1 563 804

## Description

L'invention se rattache au secteur technique des appareils médicaux et d'hygiène.

On connait des appareils conformés pour moucher les personnes, notamment les bébés. Généralement, ces appareils comprennent un embout nasal relié à un corps, assujetti à des moyens d'aspiration. A partir de cette conception de base, plusieurs solutions existent.

Dans une forme de réalisation, l'appareil comprend un embout nasal équipé intérieurement d'un filtre pour retenir les mucosités. L'embout est emmanché à l'extrémité d'un corps cylindrique dont l'autre extrémité est reliée à un tuyau d'aspiration équipé d'un embout bucal. Avec ce type d'appareil, il est nécessaire, à chaque utilisation, de changer l'embout nasal équipé du filtre. Le coût d'utilisation d'un tel appareil est donc relativement élevé.

On connaît également des appareils présentant un embout nasal emmanché dans un corps, dont une extrémité reçoit un bouchon d'obturation, en communication avec un tuyau d'aspiration équipé d'un embout bucal. Un filtre est monté dans le bouchon d'obturation.

Ce type d'appareil est divulgué dans le brevet FR-A-2562424 dont le demandeur de la présente est également titulaire. Cet appareil est conformé pour être très facilement nettoyé et ne nécessite pas de changer l'embout nasal à chaque utilisation. En outre, l'ensemble des éléments constitutifs de cet appareil sont réalisés en matière plastique transparente, ce qui permet de voir les mucosités aspirées.

Il apparait donc que l'un de ces appareils propose une solution avec embout nasal jetable à chaque utilisation, alors que l'autre propose une solution où l'embout nasal peut être le même à chaque utilisation, avec simplement la nécessité de nettoyer chaque fois l'appareil.

On connaît également par l'enseignement du brevet UK 1563804, un appareil apte à extraire les mucosités nasales. Cet appareil comprend un corps présentant à l'une de ses extrémités, un embout d'aspiration et, à l'autre extrémité, un embout nasal. Les embouts sont disposés coaxialement, en étant séparés d'une distance déterminée. Compte-tenu de l'alignement coaxial de l'embout nasal et de l'embout d'aspiration, on ne peut exclure des risques d'aspiration des mucosités, même si les embouts sont séparés. Rien n'interdit aux mucosités aspirées, de passer de l'embout nasal à l'embout d'aspiration.

Par le Brevet EP -A- 0451062, on connaît un appareil pour moucher les personnes comprenant un embout nasal emmanché de manière démontable sur un plan tubulaire formant un réservoir sur lequel est placé un produit de récupération des mucosités. L'embout nasal peut être emmanché sur un tube formé angulairement génératrice du réservoir. Le réservoir n'est pas distinct de l'embout nasal mais est constitué par l'adjonction d'un organe selon un disque rapporté sur la périphérie interne de l'embout. On obtient non pas un véritable réservoir mais tout au plus une barrière au passage des sécrétions.

Le problème que se propose de résoudre l'invention est de réaliser un appareil à moucher du type de celui décrit dans le brevet précité FR-A-2562424, avec la possibilité de jeter l'embout nasal, seulement dans des cas spécifiques, par exemple, lorsqu'il est difficile de nettoyer l'appareil. Ce peut être le cas lors de certains voyages. Ce peut être également le cas lorsque le sujet à moucher présente certaines maladies, notamment contagieuses.

Pour résoudre un tel problème et éviter aux mucosités aspirées d'aller dans le corps de l'appareil au niveau de l'embout bucal d'aspiration, il a été conçu et mis au point un appareil à moucher, conforme aux caractéristiques de la revendication 1.

L'invention est exposée, ci-après plus en détail à l'aide des dessins annexés, dans lesquels :

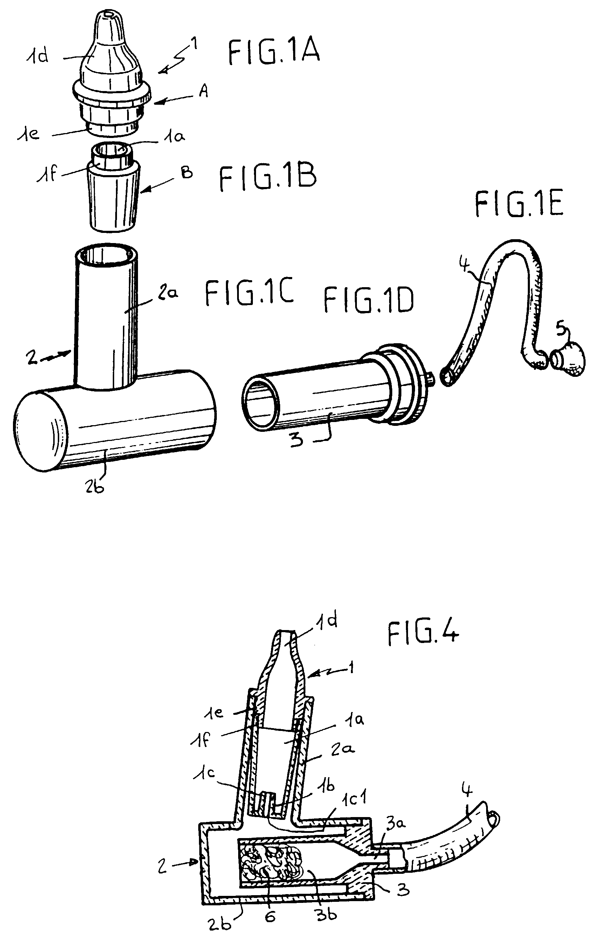

La figure 1 est une vue en perspective avant montage des principaux éléments d'un appareil à moucher, dans une forme de réalisation préférée, et équipé d'un embout nasal selon l'invention.
La figure 2 est à une échelle plus importante, une vue en coupe de l'embout nasal.
La figure 3 est une vue en coupe considérée selon la ligne 3.3 de la figure 2.
La figure 4 est une vue en coupe longitudinale de l'ensemble de l'appareil.

L'embout nasal est désigné dans son ensemble par (1).

L'embout (1) est monté sur un appareil qui comprend, de manière connue, un corps (2) recevant ledit embout nasal (1) et équipé de moyens d'aspiration. Plus particulièrement, le corps (2), de forme générale cylindrique, est ouvert à l'une de ses extrémités, pour être obturé temporairement par un bouchon (3) relié par un ajutage (3a) à un flexible (4) équipé d'un embout bucal d'aspiration (5). L'ajutage (3a) est en communication avec un chambrage interne (3b) susceptible de recevoir un organe de filtration (6).

En ce qui concerne l'embout nasal (1), ce dernier est avantageusement emmanché à force dans un tube (2a) formé angulairement par rapport aux génératrices d'un réservoir (2b) recevant le bouchon (3). Le tube (2a) est en communication avec le réservoir (2b).

Selon l'invention, l'embout nasal (1) présente un réservoir (1a) dont le fond (1b) est en communication avec le corps d'aspiration (2), au moyen d'un agencement (1c) apte à assurer la retenue des mucosités. Dans l'exemple illustré, l'embout (1) est emmanché dans le tube (2a).

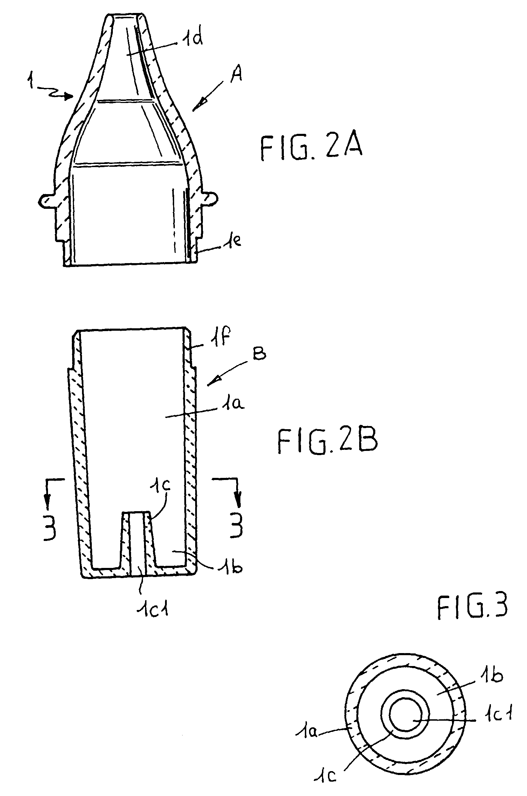

Comme le montre la figure 2, l'agencement de retenue des mucosités (1c) est formé par un bossage coaxial percé en (1c1) de part en part, pour assurer la communication entre l'embout et le corps. Ce bossage (1c) est de forme tronconique, le trou (1c1) étant cylindrique.

Avantageusement, l'embout nasal (1) est en deux parties (A) et (B). La partie (A) est profilée en (1d) pour coopérer avec une narine en constituant l'embout nasal en tant que tel. La partie (B) constitue le réservoir (1a) avec le bossage de retenue et d'aspiration (1c). Ces deux parties présentent des agencements (1e) et (1f) complémentaires pour leur emmanchement à force.

A noter que ces deux parties peuvent être rendues solidaires à demeure par collage.

Compte-tenu de ces dispositions, il apparait qu'en aspirant par l'embout bucal, les mucosités sont retenues dans le réservoir (1a) de l'embout nasal, compte-tenu du bossage coaxial d'aspiration (1c).

Dans des cas extrêmes, les mucosités sont retenues en dernier ressort, par le filtre (6) disposé dans le bouchon (3). Il est ainsi possible, si l'on ne souhaite pas nettoyer l'appareil, de jeter l'embout (1) et de le remplacer par un embout identique pour une prochaine utilisation. Par contre, à chaque utilisation, l'appareil, notamment l'embout nasal (1) peut être nettoyé totalement et réutilisé.

Comme déjà indiqué, l'ensemble des éléments constitutifs de l'appareil sont exécutés en matière plastique transparente, permettant non seulement de voir les mucosités aspirées mais également de pouvoir doser l'aspiration en fonction de la quantité de mucosités prélevées.

Les avantages ressortent bien de la description, en particulier on souligne et on rappelle la possibilité d'utiliser à volonté l'appareil, en jetant à chaque utilisation l'embout, ou bien en nettoyant l'embout à chaque utilisation permettant ainsi de le réutiliser.

## Revendications

1. Appareil à moucher comprenant un corps (2) équipé d'un embout nasal (1) et de moyens d'aspiration (3) (4) (5), le corps étant constitué d'un tube (2a) formé angulairement aux génératrices d'un réservoir (2) que reçoit coaxialement les moyens d'aspiration (3) (4) (5), caractérisé en ce que l'embout nasal présente un réservoir (1a) dont le fond (1b) est en communication avec le corps d'aspiration (2) au moyen d'un bossage coaxial (1c) percé de part en part en (1c1), pour assurer la retenue des mucosités, ledit réservoir (1a) étant emmanché dans le tube (2a) du corps (2).

2. Appareil à moucher selon la revendication 1, caractérisé en ce que le bossage (1c) est de forme tronconique.

3. Appareil à moucher selon la revendication 1, caractérisé en ce que l'embout nasal est en deux parties (A) et (B) dont l'une (A) est profilée pour coopérer avec une narine tandis que l'autre (B) présente le bossage de retenue et d'aspiration (1c).

4. Appareil à moucher selon la revendication 3, caractérisé en ce les deux parties (A) et (B) sont emmanchées à force.

5. Appareil à moucher selon la revendication 1, caractérisé en ce que les deux parties (A) et (B) sont emmanchées à force et rendues solidaires à demeure.

6. Appareil à moucher selon la revendication 1, caractérisé en ce que l'une des extrémités du réservoir (2b) du corps présente un bouchon d'obturation (3) conformé intérieurement pour recevoir ou non un organe de filtration (6) et autoriser l'aspiration au moyen d'un flexible (4) relié à un embout buccal (5).

## Claims

1. Device for removing nasal secretions, comprising a body (2) equipped with a nosepiece (1) and suction means (3), (4) and (5), the body consisting of a tube (2a) formed at an angle to the generatrices of a reservoir (2b) which receives the suction means (3), (4) and (5) coaxially, characterized in that the nosepiece has a reservoir (1a) whose bottom (1b) is in communication with the suction body (2) by way of a coaxial boss (1c) which has a passage running right through it at (1c1), to ensure retention of the mucous secretions, the said reservoir (1a) being fitted in the tube (2a) of the body (2).

2. Device for removing nasal secretions according to Claim 1, characterized in that the boss (1c) has a truncated cone shape.

3. Device for removing nasal secretions according to Claim 1, characterized in that the nosepiece is in two parts (A) and (B), of which one (A) is profiled to fit a nostril while the other (B) has the retention and suction boss (1c).

4. Device for removing nasal secretions according to Claim 3, characterized in that the two parts (A) and (B) are fitted by force.

5. Device for removing nasal secretions according to Claim 1, characterized in that the two parts (A) and (B) are fitted by force and joined permanently.

6. Device for removing nasal secretions according to Claim 1, characterized in that one of the ends of the reservoir (2b) of the body has a closure plug (3) which is shaped on the inside to optionally receive a filtration member (6) and to permit suction by way of a flexible tube (4) connected to a mouthpiece (5).

## Patentansprüche

1. Schnäuzgerät bestehend aus einem Hauptteil (2) mit Nasenaufsatz (1) und Ansaugeinrichtungen (3) (4) (5), wobei das Hauptteil aus einem Röhrchen (2a) besteht, das winkelig an den Mantellinien eines Behälters (2b) ausgebildet ist, der koaxial die Ansaugeinrichtungen (3) (4) (5) aufnimmt, dadurch gekennzeichnet, daß der Nasenaufsatz einen Behälter (1a) aufweist, dessen Boden (1b) über einen durchgehend durchbohrten (1c1) koaxialen Vorsprung (1c) mit dem Ansaugkörper (2) in Verbindung steht, um den Schleim zurückzuhalten, wobei der Behälter (1a) in das Röhrchen (2a) des Hauptteils (2) eingepaßt ist.

2. Schnäuzgerät nach Anspruch 1, dadurch gekennzeichnet, daß der Vorsprung (1c) eine kegelstumpfartige Form besitzt.

3. Schnäuzgerät nach Anspruch 1, dadurch gekennzeichnet, daß der Nasenaufsatz aus zwei Teilen (A) und (B) besteht, wobei der eine Teil (A) so profiliert ist, daß er in ein Nasenloch gesteckt werden kann, während der andere Teil den Rückhalte- und Ansaugvorsprung (1c) aufweist.

4. Schnäuzgerät nach Anspruch 3, dadurch gekennzeichnet, daß die beiden Teile (A) und (B) unter Kraftaufwand aufeinandergepreßt sind.

5. Schnäuzgerät nach Anspruch 1, dadurch gekennzeichnet, daß die beiden Teile (A) und (B) unter Kraftaufwand aufeinandergepreßt und dauerhaft fest miteinander verbunden sind.

6. Schnäuzgerät nach Anspruch 1, dadurch gekennzeichnet, daß eines der Enden des Behälters (2b) des Hauptteils einen Verschlußstopfen (3) aufweist, der innen so ausgebildet ist, um gegebenenfalls ein Filterorgan (6) aufzunehmen und die Ansaugung mittels eines mit einem Mundstück (5) in Verbindung stehenden Schlauchs (4) zu ermöglichen.
